# EUROPÄISCHE PATENTSCHRIFT

(11) **EP 0 377 782 B1**
(45) Veröffentlichungstag und Bekanntmachung des Hinweises auf die Patenterteilung: **20.09.1995**
(21) Anmeldenummer: 89112830.8
(22) Anmeldetag: 13.07.1989
(51) Int. Cl.: G01N 33/28, G01N 27/22

(54) **Verfahren zur Feststellung des Alkoholgehaltes und/oder des Heizwertes von Kraftstoffen**
Method for the determination of the alcohol content and/or the calorific value of fuels
Méthode pour la détermination de la teneur en alcool et/ou la valeur calorifique de carburants

(30) Priorität: 08.12.1988 DE 3841264
(43) Veröffentlichungstag der Anmeldung: 18.07.1990
(73) Patentinhaber: FEV Motorentechnik GmbH & Co. KG, D-52078 Aachen (DE)
(72) Erfinder: Schmitz, Günter, Dr.-Ing., D-5100 Aachen (DE); Kutz, Hans-Jürgen, Dipl.-Ing., DE-5100 Aachen (DE)
(74) Vertreter: Langmaack, Jürgen, Dipl.-Ing.

(56) Entgegenhaltungen:
- WO-A-85/01352
- DE-A- 2 544 444
- DE-C- 746 944
- US-A- 4 288 741
- US-A- 4 426 616
- IEEE TRANSACTIONS ON VEHICULAR COMMUNICATIONS, Band VT-27, Nr. 3, August 1978, New York, NY (US); J.W. HILE et al., Seiten 142-144#
- PATENT ABSTRACTS OF JAPAN, Band 005, Nr. 183 (M-097), 21. November 1981#
- PATENT ABSTRACTS OF JAPAN, Band 6, Nr. 133 (P-129)(1011), 20. Juli 1982#

## Beschreibung

Die Erfindung bezieht sich auf ein Verfahren zur Feststellung des Alkoholgehaltes und/oder des Heizwertes von Kraftstoffen durch Messung elektrisch meßbarer Größen in einer den Kraftstoff enthaltenden Meßzelle und auf eine Vorrichtung zur Ausführung des Verfahrens entsprechend dem Oberbegriff des Anspruchs 1.

In Anbetracht der langfristig nur in begrenztem Umfang verfügbaren Reserven an fossiler Energie, insbesondere der aus Rohöl gewonnenen Kraftstoffe, und in Anbetracht der steigenden Anforderungen an den Umweltschutz, wird diesen Kraftstoffen in zunehmendem Maße Methyl- oder Äthylalkohol zugemischt. Dabei soll ein beliebiges Tanken sowohl von Reinkraftstoffen als auch von Mischkraftstoffen möglich sein. Bei höheren Alkoholanteilen ist dabei die Kenntnis des Mischungsverhältnisses erforderlich, um eine optimale Arbeitsweise der Brennkraftmaschine zu erreichen und dabei insbesondere eine genaue, den Betriebsverhältnissen angepaßte Kraftstoffzumessung zu ermöglichen. Besondere Probleme bereitet dabei die laufende Feststellung des Alkoholgehaltes des der Brennkraftmaschine im Betrieb zugeführten Kraftstoffs bei Fahrzeugmotoren, bei denen durch das beliebige Tanken der Kraftstoffarten jede mögliche Mischung erreicht werden kann.

Die bekannten optischen Verfahren sind zu diesem Zweck kaum geeignet, da sie meist Grenzflächeneffekte zur Bestimmung des Brechungsindexes ausnutzen, aus dem dann auf den Alkoholanteil geschlossen werden kann. Abgesehen von der Schwierigkeit der Auswertung bei Fahrzeugmotoren ist ein Nachteil dieses Verfahrens auch, daß die messend zu beobachtende Mischung eine hohe Homogenität aufweisen muß, die insbesondere auch an der Grenzfläche vorhanden sein muß. Mit diesem Verfahren wurden nicht die erforderlichen Genauigkeiten erreicht.

Es wurde daher vorgeschlagen, den Alkoholanteil in Kraftstoffen durch eine Dielektrizitätsbestimmung festzustellen, und ein solches Verfahren hätte den Vorteil, daß die Problematik der Messung von Grenzflächeneffekten behoben ist, da die Messung volumetrisch erfolgt. Andererseits geht in die volumetrische Dielektrizitätsbestimmung in starkem Maße der Leitwert der Mischung ein (Querempfindlichkeit). Da aber der Leitwert sehr stark von Verunreinigungen oder Wasseranteilen abhängt, führt auch ein solches Meßverfahren zu unbrauchbaren Ergebnissen.

Die vorstehend als nachteilig erkannten Verfahren sind beschrieben in den Veröffentlichungen IEEE Trans. Vehicular Techn. Vol. VT-27(3) sowie Patent Abstr. of Japan, 6(133) (P-129) (1011) für die Dielektrizitätsbestimmung mittels einer Kapazitätsmessung.

Aus DE-A-25 44 444 ist es bekannt, zum Betrieb eines Kraftfahrzeuges die elektrische Leitfähigkeit des jeweils verwendeten Kraftstoffs in seiner flüssigen Phase zu messen und in Abhängigkeit von der festgestellten Leitfähigkeit das Luft/Kraftstoff-Verhältnis bei der Gemischbildung zu beeinflussen. Wie vorstehend angegeben, führen diese Verfahren bei der Bestimmung des Alkoholanteils in Kraftstoffen mit dem Ziel, einen so gewonnenen Meßwert unmittelbar im Fahrbetrieb eines Fahrzeugmotors zu verwenden, weder auf der Basis einer Dielektrizitätsbestimmung noch auf der Basis einer Leitwerbestimmung wegen der hohen Fehlerrate zu brauchbaren Ergebnissen.

In der Schrift "Proceedings of the Fourth International Symposium on Alcohol Fuels Technology", Sao Paulo, Brasilien, vom 05.10.1980, wird die Möglichkeit der Feststellung des Alkoholgehaltes von Kraftstoffen durch Dielektrizitätsmessungen beschrieben. Aufgrund der Einflüsse von Temperatur und Leitwert (hervorgerufen durch Wasseranteile oder andere Verschmutzungen im Kraftstoff) wurde das Verfahren jedoch verworfen, da eine für Verbrennungsmotoren geeignete, zuverlässige Messung nicht durchgeführt werden konnte.

Der Erfindung liegt daher die Aufgabe zugrunde, ein Verfahren der eingangs bezeichneten Art zu schaffen, das insbesondere in der Anwendung auf Fahrzeugmotoren eine genaue und zuverlässige Feststellung des Alkoholanteils an Kraftstoffen erlaubt, um die gewonnen Meßgrößen im Betrieb zur Regelung der Kraftstoffzufuhr verwenden zu können.

Diese Aufgabe wird mit den Mitteln des Kennzeichungsteiles des Anspruchs 1 gelöst.

Da die relative Dielektrizitätszahl des Kraftstoffgemisches mit der Temperatur schwankt, lassen sich genauere Bestimmungen erzielen, wenn neben der Kapazitätsmessung auch die Temperatur erfaßt wird. und dann aus diesen Größen gemeinsam der Alkoholgehalt und/oder der Heizwert bestimmt wird. Gleiches gilt für den Leitwert, der ebenfalls mit der Temperatur schwankt und daher in Zusammenhang mit dieser gemessen und ausgewertet werden sollte. Für eine Verwendung zur Regelung im Betrieb lassen sich zuverlässige Werte ist dann erzielen und eine höhere Genauigkeit erreichen, wenn die gegebenenfalls fehlerbehafteten Meßgrößen für die Dielektrizitätszahl bzw. Kapazität, Leitwert und Temperatur mit in die Bestimmung des Alkoholgehaltes und/oder des Heizwertes einbezogen und durch gemeinsame rechnerische oder schaltungstechnische Verknüpfung ausgewertet werden.

Besondere Vorteile lassen sich erzielen, wenn die Verknüpfung der Meßwerte in einem programmierbaren Rechenwerk (Prozessor) erfolgt, da sich beliebige Arten von Verknüpfungen mit relativ geringem Aufwand realisieren lassen. So kann beispielsweise eine formelmäßige Verknüpfung (Multiplikation, Addition etc.) oder auch eine tabellarische (bzw. kennfeldmäßige) Verknüpfung recht einfach durchgeführt werden.

Bei der Fertigung der erforderlichen elektronischen Gesamtschaltung (Meß- und Weiterverarbeitungsschaltung) ist für jedes produzierte Exemplar ein separater Abgleich zur Korrektur von Bauteiltoleranzen oder auch zur Abstimmung auf spezifische Anforderungen notwendig. Zu diesem Zweck wird häufig ein Abgleich von einem oder mehreren elektronischen Bauteilen, wie z.B. Kondensatoren, vorgenommen.

Bei Ausführungen, die einen Prozessor enthalten, kann der Aufwand durch Verwendung von elektronisch programmierbaren Bausteinen, wie z.B. PROM oder EPROM, reduziert werden, indem die Anpassung jedes Exemplars durch Einprogrammieren von entsprechenden Parametern durchgeführt wird (Band-Ende-Programmierung). Dies erfordert allerdings das Vorhandensein solcher einzeln programmierbarer Bausteine, deren Kosten deutlich über denen eines z.B. maskenprogrammierten Bausteins liegen.

Gemäß einem weiteren vorteilhaften Merkmal der Erfindung ist daher vorgesehen, daß wenigstens einem Prozessor wenigstens ein für die jeweilige Anwendung erforderlicher Parameter aufgeprägt wird, indem wenigstens ein Widerstand eingestellt wird, dessen Einstellung durch einen Prozessor über einen Analog/Digital-Umsetzer eingelesen wird.

Mit Hilfe eines besonders einfachen Widerstandsabgleichs ist somit eine "Programmierung" von Parametern möglich, auf die der Prozessor ähnlich wie bei der PROM-Lösung zugreifen kann.

Der Vorteil eines solchen Vorgehens liegt darin, die sehr flexible Band-Ende-Programmierung auch bei ausschließlicher Verwendung von festen Programmspeichern, wie maskenprogrammierbaren ROMs, zu ermöglichen.

Besondere Vorteile sind erreichbar, wenn die Messung der Kapazität nicht in einer LC-Schaltung, die aufwendig und störungsempfindlich sein kann, durchgeführt wird, sondern vielmehr in einer RC-Schaltung, die jedoch einen starken Quereinfluß zum Leitwert aufweist. Durch ebenfalls erfolgende Messung des Leitwertes kann jedoch der Einfluß des Leitwertes aus der Kapazitätsmessung z.B. rechnerisch oder durch Verwendung einer Tabelle (z.B. innerhalb eines Mikroprozessors), eliminiert werden.

Hinsichtlich weiterer vorteilhafter Merkmale der Erfindung wird auf die Ansprüche, die Zeichnungen und die nachfolgende Beschreibung Bezug genommen.

Ausführungsbeispiele der Erfindung werden anhand der Zeichnungen beschrieben.

Fig. 1 zeigt im Diagramm die Dielektrizitätszahl als Funktion des Leitwertes G in Abhängigkeit von dem Wassergehalt und dem Methanolgehalt des Kraftstoffs.

Fig. 2 zeigt ein bevorzugtes Beispiel einer Meßzelle zur Ausführung des Verfahrens gemäß der Erfindung.

Fig. 3 zeigt schematisch eine Anordnung zur Anwendung des Verfahrens gemäß der Erfindung zur Steuerung bzw. Regelung einer Einspritzbrennkraftmaschine.

Fig. 4 zeigt eine Kippschaltung als bevorzugtes Ausführungsbeispiel einer Schaltung zur Ausführung des Verfahrens gemäß der Erfindung.

Fig. 5 zeigt schematisch eine als LC-Schwingkreis ausgebildete Schaltung zur Kapazitätsmessung bei der Ausführung des Verfahrens gemäß der Erfindung.

Fig. 6 zeigt eine Schaltung, welche einen Abgleich zur Korrektur von Bauteiltoleranzen oder auch zur Abstimmung auf spezifische Anforderungen ermöglicht.

Fig. 7 und 8 zeigen Schaltungen der vorgenannten Art, welche zur Messung der Dielektrizitätszahl eines unbekannten Mediums geeignet sind.

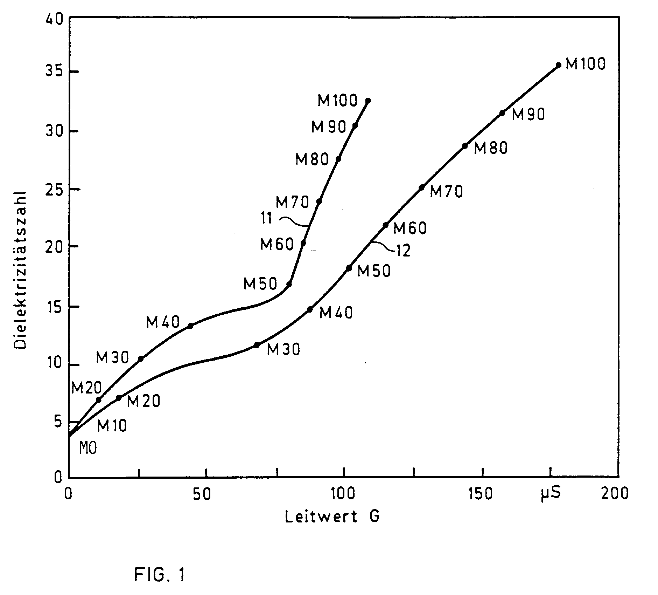

In der Darstellung gemäß Fig. 1 sind die Dielektrizitätszahlen in der Ordinate und die Leitwerte G in der Abszisse aufgetragen. Linienzug 11 zeigt die Abhängigkeit der Dielektrizitätszahlen von dem Anteil der Methanolbeimischung bei einem Wasseranteil von 0% im Kraftstoff, während Linienzug 12 die entsprechenden Werte bei einem Anteil von 2,5% H O darstellt. Auf den Linienzügen sind die jeweiligen Meßpunkte für variable Methanolanteile von 0% bis 100% (MO bis M100) aufgetragen.

Man erkennt, daß bei höheren Wasseranteilen bestimmte Dielektrizitätswerte bei höheren Leitfähigkeiten gemessen werden. Empirisch ermittelte Kurvenscharen der in Fig. 1 dargestellten Art ermöglichen bei einer kombinierten Messung von Leitfähigkeit und Dielektrizitätszahl eine Korrektur der Dielektrizitätsmessung durch die Leitwertbestimmung.

Durch die Kenntnis des Leitwerts können nun die Quereinflüsse auf die Kapazitätsmessung durch Verunreinigungen des Kraftstoffes auskorrigiert werden. Wie Fig. 1 zeigt, wirkt beispielsweise ein höherer Wasseranteil im Kraftstoff kapazitätserhöhend. Durch Messung des Leitwerts kann diese Erhöhung der Kapazität bei der Bestimmung des Alkoholanteils berücksichtigt werden.

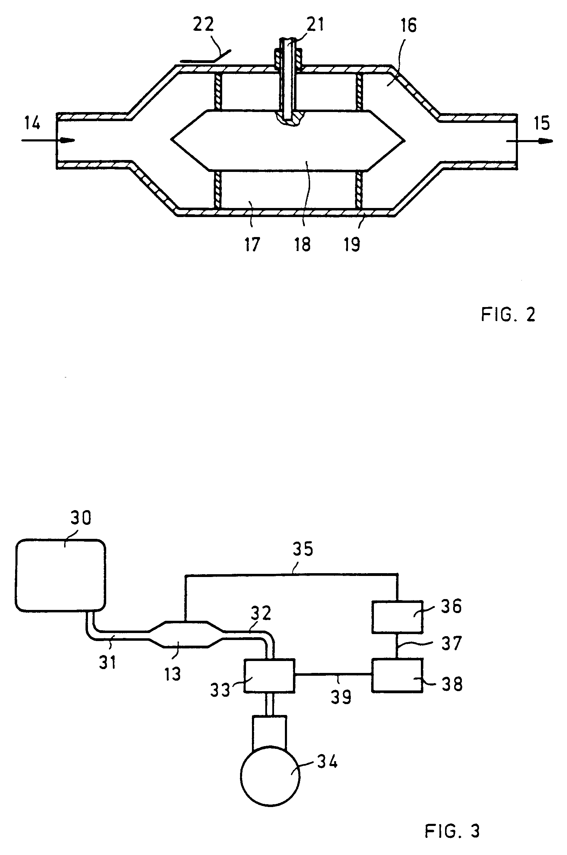

Fig. 2 zeigt eine bevorzugte Ausführungsform einer Meßzelle zur Ausführung des Verfahrens gemäß der Erfindung. In die Meßzelle gelangt der Kraftstoff über Zufluß 14, und er verläßt die Meßzelle über Abfluß 15. Der Kraftstoffstrom teilt sich in der flächenhaften Darstellung der Fig. 2 auf in Strömungswege 16 und 17, die durch einen Mittelzylinder 18 gebildet werden. Wenn der Mittelzylinder 18 und der Außenmantel 19 ganz oder teilweise elektrisch leitend sind, können diese Wandungen bzw. Wandungsteile der Meßzelle die Elektroden des frequenzbestimmenden Kondensators einer Meß- bzw. Auswerteschaltung darstellen. Innerhalb der wenigstens teilweise leitfähigen Wandung der Meßzelle, die die erste Elektrode darstellt, ist dann der wenigstens teilweise leitfähige Mittelzylinder 18 als Strömungskörper die zweite Elektrode.

Der Mittelzylinder 18 bildet zusammen mit dem Außenmantel 19 den eigentlichen Meßkondensator, der das Meßvolumen umschließt. An Anschlüssen 21 und 22 können die entsprechenden Werte abgegriffen werden, und zwar sowohl für die Kapazität als auch für den Leitwert an denselben Elektroden. Die abgegriffenen Werte werden dann zur weiteren Verarbeitung in die entsprechenden Schaltung eingegeben.

Wie aus der schematischen Darstellung der Fig. 3 hervorgeht, gelangt mit Alkoholanteilen gemischter Kraftstoff aus Kraftstofftank 30 über eine Leitung 31 zur Meßzelle 13 und von dort über eine Leitung 32 zu einer Zumeßeinrichtung 33, die im Regelfall eine Einspritzpumpe mit entsprechenden Einspritzdüsen ist. Der Kraftstoff wird in direkter oder indirekter Einspritzung der Verbrennung in Motor 34 zugeführt.

Die in der Meßzelle 13 abgegriffenen Werte der Kapazität und des Leitwertes werden über Meßleitung 35 einer Auswerteschaltung oder Auswerteeinheit 36 zugeführt. Vorzugsweise ist die Schaltung 36 schwingungsfähig, und ihre Ausgangsfrequenz kann dann als Maß der Kapazität ausgewertet werden. In dieser schwingungsfähigen Schaltung 36 ist die Kraftstoffmenge in Meßzelle 13 oder ein Teil davon das Dielektrikum des kapazitiven Teils der Auswerteschaltung 36.

Die von der Auswerteschaltung 36 abgegebenen Signale gelangen über eine Leitung 37 zu einem Einspritzrechner 38, und dieser steuert über eine Leitung 39 die Zumeßeinrichtung 33.

Der jeweils in der Meßzelle 13 gemessene und in der Auswerteeinheit 36 ausgewertete Mischungsanteil wird also betrieblich nicht verändert, sondern es wird vielmehr die Art der Einspritzung je nach den gemessenen und ausgewerteten Werten geändert. Gemäß einer bevorzugten Ausführungsform kann bei der Steuerung bzw. Regelung von Einspritzbrennkraftmaschinen die Messung des Alkoholanteils des zugeführten Kraftstoffs zur Vorsteuerung der Einspritzmenge dienen, während die Feinregelung des Luftverhältnisses über eine Lambdaregelung bekannter Art erfolgt. Insbesondere bei Kraftfahrzeugmotoren kann es dabei zweckmäßig sein, daß bei der Anwendung des Verfahrens gemäß der Erfindung auf Einspritzbrennkraftmaschinen die Schaltung 36 als Auswerteeinheit in das Einspritzsystem schaltungs- und programmtechnisch integriert ist. Weitere Vorteile können verfahrenstechnisch dadurch erreicht werden, daß zusätzlich die Temperatur des in der Meßzelle 13 befindlichen Kraftstoffs festgestellt und in die Auswerteeinheit zur Kompensation des Temperatureinflusses eingegeben wird.

Besondere Vorteile sind erreichbar, wenn die Schaltung 36 eine Kippschaltung ist, in der die Kraftstoffmenge in der Meßzelle das Dielektrikum des frequenzbestimmenden Kondensators bildet. Dabei wird vorzugsweise die Kippfrequenz der Kippschaltung zur Bestimmung der Kapazität ausgewertet.

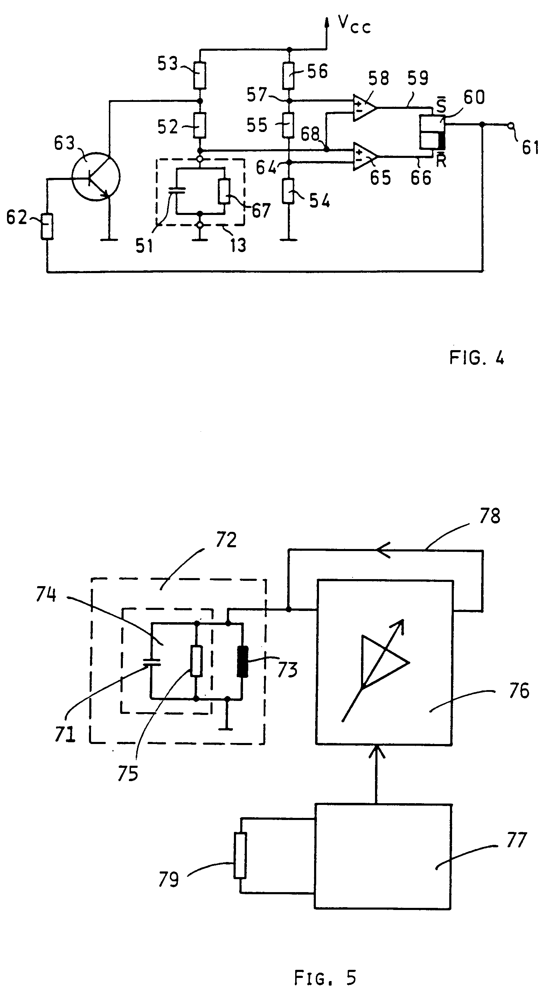

Eine bevorzugte Ausführungsform einer Kippschaltung der erwähnten Art ist in Fig. 4 dargestellt. Die Kapazität 51 der Meßzelle 13 wird über Widerstände 52 und 53 von der Speisespannung V so lange aufgeladen, bis eine Schwellenspannung U1, die durch Widerstände 54, 55 und 56 an Punkt 57 gebildet wird, an Komparator 58 überschritten wird. Komparatorausgang 59 geht dann von "High"- auf "Low-Pegel" über und setzt Flip-Flop 60, dessen Ausgang 61 somit auf "High-Pegel" schaltet. Über Widerstand 62 wird nun ein Transistor 63 eingeschaltet, der den Entladevorgang der Kapazität 51 über den Widerstand 52 einleitet. Wenn nun die Spannung an der Kapazität 51 unter einen Schwellenwert U2 sinkt, der durch die Widerstände 54, 55 und 56 an Punkt 64 gebildet wird, so schaltet ein Komparator 65 seinen Ausgang 66 von "High"-auf "Low-Pegel". Dadurch wird Flip-Flop 60 zurückgesetzt; der Transistor 63 schaltet in den hochohmigen Zustand, und die Kapazität 51 wird erneut geladen.

Um eine sichere Funktion der in Fig. 4 dargestellten Schaltung auch bei großen Leitwerten zu erreichen, kann zwischen den Widerstand 52 und die Kapazität 51 mit Leitwertfunktion 67 der Meßzelle 13 eine Koppelkapazität zur Gleichstromunterdrückung eingebaut werden, wobei der Anschluß 68 zu den Komparatoren 58 und 65 zwischen Widerstand 52 und der Koppelkapazität liegen muß.

In einem zweiten Schaltungsteil oder auch in derselben Schaltung erfolgt die Messung des Leitwertes. Dabei kann dieselbe Frequenz wie für die Kapazitätsmessung verwendet werden, oder auch eine andere im Wert stark abweichende Frequenz, die beispielsweise in einem zweiten Oszillator erzeugt wird, um Einflüsse der Frequenz auf die Leitwertmessung zu unterbinden. Jedoch kann auch gerade der Frequenzeinfluß auf die Leitwertmessung benutzt werden, um eine genauere Bestimmung des Alkoholgehaltes oder des Heizwertes zu ermöglichen.

Diese Messung des Leitwertes kann über eine zweite Elektrodenanordnung erfolgen oder auch über dieselben Elektroden vorgenommen werden, die auch zur Kapazitätsmessung verwendet werden. Da sich bei Verwendung nur einer Elektrodenanordnung produktionstechnische Vorteile ergeben, ist auch ein größerer Schaltungsaufwand gerechtfertigt, um die Auswertung vorzunehmen. So kann z.B. die Meßelektrode zeitlich nacheinander zur Kapazitätsmessung und zur Leitwertmessung benutzt werden.

Dabei kann es vorteilhaft sein, daß die Messung von Kapazität und Leitwert des Kraftstoffs über gemeinsame Elektroden erfolgt, jedoch verschiedene Meßschaltungen benutzt werden, die durch Verwendung stark unterschiedlicher Frequenzen sich gegenseitig nicht beeinflussen.

Eine andere Schaltungsvariante besteht darin, daß ein LC-Schwingkreis zur Messung der Kapazität verwendet wird. Besondere Vorteile bietet dieses Verfahren, weil die gemessene Frequenz primär unabhängig von dem Leitwert zwischen den Elektroden ist. Bei größeren Leitwerten, bedingt z.B. durch Zusatzstoffe oder Verunreinigungen im Kraftstoff, ist es jedoch problematisch, die Funktion der Schaltung sicherzustellen. Hierbei kann wiederum die zusätzliche Messung des Leitwertes sinnvoll sein, die eine Anpassung der Schwingschaltung an den gemessenen Leitwert bewirkt.

Eine bevorzugte Ausführungsform ist in Fig. 5 dargestellt. Meßzelle 74 bildet den Kondensator 71 eines Schwingkreises 72, der als anderen frequenzbestimmenden Teil über eine Spule 73 verfügt. Als unerwünschtes Element wirkt der Leitwert 75, der parallel zum Schwingkreis liegt und u.a. durch die in dem Kraftstoff vorhandenen Zusatzstoffe oder Verunreinigungen verursacht wird. In einer den Schwingkreis über eine Rückführung 78 entdämpfenden Verstärkerschaltung 76 wird die Verstärkung in Abhängigkeit des gemessenen Leitwertes 79 eingestellt, um einen Betrieb des Schwingkreises auch bei großen Leitwerten sicherzustellen. Die Messung des Leitwertes kann in einer getrennten Schaltung 77 erfolgen, wie in Fig. 5 dargestellt ist. Dabei kann entweder die zur Kapazitätsmessung benutzte Elektrode oder eine getrennte Elektrodenanordnung verwendet werden. Jedoch kann auch die Messung in derselben Schaltung erfolgen, in der die Erzeugung der Schwingung erfolgt, z.B. indem die Amplitude der Schwingung bestimmt wird, die ein Maß für die Größe des Leitwertes darstellt. Abhängig von der gemessenen Amplitude kann eine Nachregelung der Amplitude erfolgen, damit die Funktion der Schaltung bei beliebigen Werten des Leitwertes sichergestellt wird.

Bei der Messung mit Hilfe eines LC-Schwingkreises ergeben sich bei Verwendung einer konventionellen Induktivität Probleme hinsichtlich des Bauraumes, der Störfestigkeit usw. Bei Ersetzen der Spule durch eine elektronisch simulierte Induktivität entfällt dieser Nachteil. Die Induktivität kann beispielsweise mit Hilfe eines Gyrators und eines Kondensators oder besonders vorteilhaft durch eine sogenannte "Switched Capacitor"-Schaltung realisiert werden.

Bei Verwendung einer "Switched-Capacitor"-Schaltung kann sogar die Größe der Induktivität elektronisch eingestellt werden und dadurch z.B. auch bei sich ändernden Kapazitäten des Meßkondensators die Frequenz auf einen konstanten Wert geregelt werden, was insbesondere für die Reproduzierbarkeit der Messungen von Bedeutung ist.

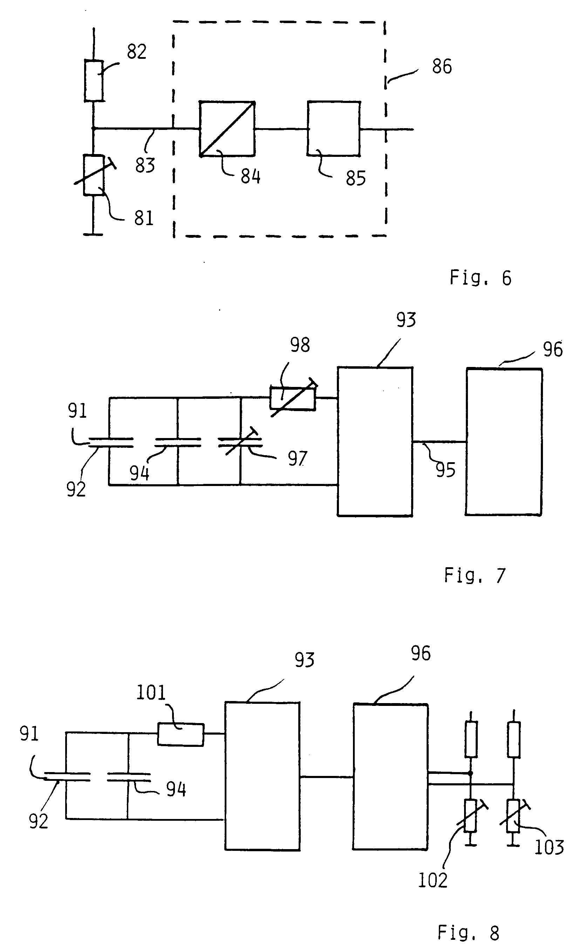

Wie Fig. 6 zeigt, wird von Widerständen 81 und 82 ein Spannungsteiler gebildet, dessen Ausgangsspannung auf Leitung 83 über einen Analog/Digital-Umsetzer 84 einem Prozessor 85 zum Einlesen weitergegeben wird.

Besondere Vorteile bezüglich der kostengünstigen Realisierung sind erreichbar, wenn der Analog-Digital-Umsetzer 84 mit dem Prozessor 85 zusammen in einem Mikro-Controller 86 integriert ist.

Vorteile gegenüber einem direkten Abgleich der elektrischen Schaltung lassen sich auch erzielen, wenn durch eine formelmäßige Berücksichtigung der eingestellten Parameter ein aufwendiger iterativer Einstellvorgang entfällt, oder wenn hierdurch eine aufwendige Einstellung mittels Trimmkondensator durch den Widerstandsabgleich ersetzt werden kann.

Beispielsweise ist in Figur 7 eine Schaltung zur Messung der Dielektrizitätszahl eines unbekannten Mediums dargestellt, wobei das Medium das Dielektrikum 91 eines Kondensators 92 bildet, der das frequenzbestimmende Glied in einem RC-Schwingkreis 93 ist. Bedingt durch Fertigungsstreuungen schwankt sowohl die von der Dielektrizitätszahl abhängige Kapazität 92 als auch die von der Dielektrizitätszahl unabhängige parasitäre Kapazität 94. Um nun einen Abgleich der Ausgangsfrequenz auf Leitung 95, die einer Weiterverarbeitungsschaltung 96 zugeführt wird, über den gesamten Meßbereich vornehmen zu können, ist ein Abgleich mit Hilfe von zwei einstellbaren Komponenten, nämlich einem Parallelkondensator 97 und einem Trimmwiderstand 98, erforderlich. Der Abgleich kann nur über einen iterativen Prozeß erfolgen; das bedeutet, daß die Meßkapazität wechselweise mit Medien verschiedener Dielektrizitätszahl befüllt werden muß und jeweils einen Abgleich des Kondensators 97 bei Vorhandensein einer niedrigen Dielektrizitätszahl und Widerstandstrimmer 98 bei Vorhandensein eines Mediums mit hoher Dielektrizitätszahl eingestellt werden muß, und zwar so lange, bis keine deutliche Verbeserung der Einstellung mehr erzielt werden kann.

In der Ausführungsform gemäß Figur 8 kommt das Verfahren gemäß der Erfindung zur Anwendung. Hierbei kann der Trimmkondensator 97 (Fig. 7) entfallen und der Trimmwiderstand 98 (Fig. 7) in einen Festwiderstand 101 umgewandelt werden. Der Abgleich erfolgt nun in der Weise, daß erstens eine Frequenzmessung mit einem Medium niedriger Dielektrizitätszahl (z.B. Luft) und zweitens eine zweite Messung mit einem Medium besonders hoher Dielektrizitätszahl durchgeführt wird. Das Ergebnis der Frequenzmessung wird durch Trimmung von zwei Widerständen 102 und 103 dem Prozessor eingegeben, so daß er dann bei zukünftigem Betrieb durch eine entsprechende formelmäßige Berücksichtigung eine Kompensation der Serienstreuung vornehmen kann.

Für die Großserienfertigung sind besondere Vorteile erzielbar durch den Einsatz eines Widerstands-Laser-Trimmverfahrens, das bei der konventionellen Technik aus zwei Gründen nicht eingesetzt werden könnte. Zum einen gibt es keine sinnvolle Möglichkeit, einen Kondensator durch Lasertrimmung abzugleichen und zum anderen ist verfahrensbedingt bei der Lasertrimmung ein iterativer Abgleich nicht möglich (Abgleich durch teilweises Wegbrennen der Widerstandsbahnen).

Ebenfalls sehr vorteilhaft kann das der Erfindung zugrunde liegende Verfahren verwendet werden, um für jedes Exemplar einer Serie eine Anpassung an den spezifischen Anwendungsfall vorzunehmen. So kann z.B. durch Kodieren mit verschiedenen Analogwerten eine Auswahl von Ausgangskennlinien vorgenommen Werden (z.B. Spannung proportional zum Alkoholanteil oder Spannung proportional zur erforderlichen Verlängerung der Einspritzzeit).

Weiterhin läßt sich ein einzeln programmierbarer Baustein einsparen für eine Programmierung einer Seriennummer, Chargennummer, einer Produkttypenkennzeichnung oder für die Realisierung eines Softwareschutzes, indem die Parameter wiederum durch Widerstandstrimmung eingestellt und über A/D-Umsetzer dem Prozessor zur Verfügung gestellt werden.

Besonders vorteilhaft ist der Einsatz dieser gesamten Verfahren in der Hybridtechnik, wo insbesondere die Lasertrimmung zum Tragen kommt. Solche Hybridschaltungen können besonders vorteilhaft in die Gehäuse von Sensoren integriert werden, die durch Kompensation der Typenstreuung und z.B. Anpassung der Ausgangskennlinie des Sensors durch den Prozessor an die spezifische Anwendung ein unmittelbar weiterverwendbares Signal zur Verfügung stellen.

Die Erfindung ist nicht auf die dargestellten und beschriebenen Ausführungsbeispiele beschränkt. So kann es vorteilhaft sein, daß zusätzlich zu von der stofflichen Beschaffenheit des Kraftstoffs abhängigen Größen von der stofflichen Beschaffenheit des Kraftstoffs unabhängige Größen gemessen und ausgewertet werden, die die stoffliche Beschaffenheit des Kraftstoffs beeinflussen. Als von der stofflichen Beschaffenheit des Kraftstoffs unabhängige Größe kann dabei die Temperatur des Kraftstoffs gemessen und zur Korrektur des ermittelten Alkoholgehaltes und/oder Heizwertes ausgewertet werden.

Die Messung der Kapazität kann nicht nur durch eine schwingfähige Schaltung erfolgen, in der in der Meßzelle enthaltener Kraftstoff Teil eines frequenzbestimmenden Kondensators ist, sondern auch dadurch, daß die Lade- und/oder die Entladezeit eines in der Meßzelle gebildeten Kondensators, dessen Dielektrikum Kraftstoff ist, zur Bestimmung des Alkoholgehaltes und/oder des Heizwertes benutzt wird.

Eine weitere vorteilhafte Möglichkeit besteht darin, daß die Messung elektrisch meßbarer Größen in einer Schaltung erfolgt, die in unmittelbarer Nähe der Meßzelle angeordnet ist, während die Weiterverarbeitung der von der Meßschaltung abgegebenen Signale in einer weiter entfernten Schaltung erfolgt.

Auch können alle oder ein Teil der von der Meßschaltung erzeugten Signale in einem kombinierten Signal zusammengefaßt und über eine Signalleitung der Weiterverarbeitungsschaltung zugeführt werden.

## Patentansprüche

1. Verfahren zur Feststellung des Alkoholgehaltes und/oder des Heizwertes von Alkohol enthaltenden Kraftstoffen durch Messung elektrisch meßbarer, von der stofflichen Beschaffenheit des Kraftstoff abhängiger Größen und von der stofflichen Beschaffenheit des Kraftstoffs unabhängiger Größen in einer den Kraftstoff enthaltenden Meßzelle, wobei als eine stoffabhängige Größe die Dielektrizätatszahl und als stoffunabhängige Größe die Temperatur als elektrisch meßbare Größen erfaßt werden und durch rechnerische oder schaltungstechnische Verknüpfung der gemessenen Größen ein Maß für den Alkohol und/oder den Heizwert des Kraftstoffe ermittelt wird, dadurch gekennzeichnet, daß in der Meßzelle als weitere stoffabhängige Größe durch eine Messung der Leitwert des Kraftstoffs bestimmt und aus allen gemessenen Größen gemeinsam der Alkoholgehalt und/oder der Heizwert des Kraftstoffs bestimmt wird.

2. Verfahren nach Anspruch 1, dadurch gekennzeichnet, daß die Dielektrizitätszahl bzw. die Kapazität durch eine Schaltung bestimmt wird, die eine besondere Querempfindlichkeit zum Leitwert aufweist, derart, daß durch die Verknüpfung mit dem Leitwert auch für Kraftstoffe mit Zusätzen oder Verunreinigungen ein zutreffender Wert für den Alkoholgehalt und/oder den Heizwert ermittelt wird.

3. Verfahren nach Anspruch 1 oder 2, dadurch gekennzeichnet, daß die Messung der Dielektrizitätszahl bzw. der Kapazität durch eine schwingfähige Schaltung erfolgt, in der in der Meßzelle enthaltener Kraftstoff Teil eines frequenzbestimmenden Kondensators ist und daß das Tastverhältnis oder einer der beiden Schaltzustände der schwingfähigen Schaltung zur Bestimmung des Leitwertes ausgewertet wird.

4. Verfahren nach einem der Ansprüche 1-3, dadurch gekennzeichnet, daß die Lade- und/oder die Entladezeit eines in der Meßzelle gebildeten Kondensators, dessen Dielektrikum Kraftstoff ist, zur Bestimmung des Alkoholgehaltes und/oder des Heizwertes benutzt wird.

5. Verfahren nach einem der Ansprüche 1-4, dadurch gekennzeichnet, daß elektrisch meßbare Größen zeitlich nacheinander gemessen werden.

6. Verfahren nach Anspruch 5, dadurch gekennzeichnet, daß für die Messung der Dielektrizitätszahl bzw. der Kapazität und des Leitwerts eine gemeinsame Elektrode zeitlich nacheinander benutzt wird.

7. Verfahren nach einem der Ansprüche 1-6, dadurch gekennzeichnet, daß die Messung elektrisch meßbarer Größen in einer Schaltung erfolgt, die in unmittelbarer Nähe der Meßzelle angeordnet ist, während die Weiterverarbeitung der von der Meßschaltung abgegebenen Signale in einer weiter entfernten Schaltung erfolgt.

8. Verfahren nach Anspruch 7, dadurch gekennzeichnet, daß alle oder ein Teil der von der Meßschaltung erzeugten Signale in einem kombinierten Signal zusammengefaßt und über eine Signalleitung, über die auch die Spannungsversorgung erfolgen kann, der Weiterverarbeitungsschaltung zugeführt werden.

9. Verfahren nach einem der Ansprüche 1-8, dadurch gekennzeichnet, daß die Messung von Kapazität und Leitwert des Kraftstoffs über gemeinsame Elektroden erfolgt, jedoch verschiedene Meßschaltungen benutzt werden, die durch Verwendung stark unterschiedlicher Frequenzen sich gegenseitig nicht beeinflussen.

10. Verfahren nach einem der Ansprüche 1-9, dadurch gekennzeichnet, daß die Messung der Kapazität des Kraftstoffs durch einen LC-Schwingkreis erfolgt, der abhängig von der Größe des Leitwerts entdämpft wird.

11. Verfahren nach Anspruch 10, dadurch gekennzeichnet, daß der L-Wert anstelle einer konventionellen Induktivität durch eine elektronische Schaltung simuliert wird.

12. Verfahren nach einem der Ansprüche 1-11, dadurch gekennzeichnet, daß bei Verwendung von Wechselspannung im Meßvorgang die Meßfrequenz bei sich ändernder Kapazität konstant gehalten wird.

13. Verfahren nach einem der Ansprüche 1-12, dadurch gekennzeichnet, daß die Verknüpfung der Größen in einem programmierbaren Rechenwerk (Prozessor) erfolgt und daß wenigstens einem Prozessor wenigstens ein für die jeweilige Anwendung erforderlicher Parameter aufgeprägt wird, indem wenigstens ein Widerstand eingestellt wird, dessen Einstellung durch einen Prozessor über einen Analog/Digital-Umsetzer eingelesen wird.

14. Verfahren nach Anspruch 13, dadurch gekennzeichnet, daß wenigstens ein Parameter zur Kompensation von Serientoleranzen verwendet wird.

15. Verfahren nach Anspruch 13, dadurch gekennzeichnet, daß wenigstens ein Parameter zur Anpassung an unterschiedliche Anwendungen verwendet wird.

16. Verfahren nach Anspruch 13, dadurch gekennzeichnet, daß wenigstens ein Parameter zur Kennzeichnung der Schaltung verwendet wird, z.B. der Seriennummer, der Chargennummer, der Produkttype oder des Softwareschutzes.

17. Verfahren nach einem der Ansprüche 13-16, dadurch gekennzeichnet, daß wenigstens ein Parameter formelmäßig verarbeitet wird.

## Claims

1. Method of determining the alcohol content and/or the calorific value of alcohol-containing fuels by measuring electrically measurable values dependent on the material composition of the fuel and values not dependent on the material composition of the fuel in a measuring cell containing the fuel, wherein as a material-dependent value the dielectric constant, and as a non-material-dependent value the temperature are measured as electrically measurable values, and by linking the values measured by electronics or circuitry, a measure is obtained for the alcohol and/or the calorific value of the fuel, characterised in that in the measuring cell, as a further material-dependent value by measurement, the conductance of the fuel is determined, and from all the values measured the alcohol content and/or calorific value of the fuel is determined.

2. Method according to claim 1, characterised in that the dielectric constant or capacitance is determined by a circuit having a particular transverse sensitivity to the conductance, such that by linking with the conductance a correct value for the alcohol content and/or the calorific value is detected even for fuels containing additives or impurities.

3. Method according to claim 1 or 2, characterised in that the measurement of the dielectric constant or capacitance is carried out by a resonant circuit, in which fuel contained in the measuring cell is part of a frequency-determining capacitor, and in that the mark-space ratio or one of the two switching states of the resonant circuit is evaluated in order to establish the conductance.

4. Method according to one of claims 1-3, characterised in that the charging and/or discharging time of a capacitor formed in the measuring cell whose dielectric is fuel is used to determine the alcohol content and/or the calorific value.

5. Method according to one of claims 1-4, characterised in that electrically measurable values are measured consecutively in time.

6. Method according to claim 5, characterised in that for measuring the dielectric constant or capacitance and the conductance a common electrode is used consecutively in time.

7. Method according to one of claims 1-6, characterised in that the measurement of electrically measurable values is carried out in a circuit disposed in the immediate vicinity of the measuring cell, whilst the subsequent processing of the signals transmitted by the measuring circuit is carried out in a more remote circuit.

8. Method according to claim 7, characterised in that all or part of the signals generated by the measuring circuit are combined into one signal and are fed via a signal lead, which may also carry the power supply, to the processing circuit.

9. Method according to one of claims 1-8, characterised in that the measurement of capacitance and conductance of the fuel is effected via common electrodes, but different measuring circuits are used which do not generate mutual interference if very different frequencies are used.

10. Method according to one of claims 1-9, characterised in that the measurement of the capacitance of the fuel is effected by an LC resonant circuit, which changes flux as a function of the level of conductance.

11. Method according to claim 10, characterised in that the conductance rather than a conventional inductance is simulated by an electronic circuit.

12. Method according to one of claims 1-11, characterised in that if alternating voltage is used in the measuring operation, the measuring frequency is kept constant whilst the capacitance varies.

13. Method according to one of claims 1-12, characterised in that the linking of values is carried out in a programmable processor, and in that at least one parameter necessary for the respective application is impressed on at least one processor, at least one resistance being set, its setting being read in by a processor via an analog-digital converter.

14. Method according to claim 13, characterised in that at least one parameter is used to compensate for serial variations.

15. Method according to claim 13, characterised in that at least one parameter is used for adapting to different applications.

16. Method according to claim 13, characterised in that at least one parameter is used to identify the circuit, e.g. the serial number, batch number, product type or software protection.

17. Method according to at least one of claims 13-16, characterised in that at least one parameter is processed by way of a formula.

## Revendications

1. Procédé pour la détermination de la teneur en alcool et/ou de la valeur calorifique de carburants contenant de l'alcool, par la mesure, dans une cellule de mesure contenant le carburant, de grandeurs électriquement mesurables dépendantes de la nature des constituants du carburant et indépendantes de la nature des constituants du carburant, les grandeurs électriquement mesurables déterminées étant la constante diélectrique comme grandeur dépendante des constituants et la température comme grandeur indépendante des constituants, et une mesure de la teneur en alcool et/ou de la valeur calorifique du carburant étant obtenue en combinant par le calcul ou par une technique de montage les grandeurs mesurées, **caractérisé** en ce qu'on détermine, par une mesure dans la cellule de mesure, la conductance du carburant comme grandeur supplémentaire dépendante des constituants, et on détermine conjointement la teneur en alcool et/ou la valeur calorifique du carburant à partir de toutes les grandeurs mesurées, conjointement.

2. Procédé selon la revendication 1, **caractérisé** en ce qu'on détermine la constante diélectrique ou encore la capacitance par un montage qui présente une sensibilité croisée particulière à la conductance, de telle sorte que la combinaison avec la conductance permet de déterminer une valeur pertinente pour la teneur en alcool et/ou la valeur calorifique même pour des carburants contenant des additifs ou des impuretés.

3. Procédé selon la revendication 1 ou 2, **caractérisé** en ce que la mesure de la constante diélectrique ou encore de la capacitance s'effectue par un montage oscillant dans lequel le carburant contenu dans la cellule de mesure fait partie d'un condensateur déterminant la fréquence, et en ce que, pour déterminer la conductance, on évalue le rapport d'impulsions ou un des deux états de commutation du montage oscillant.

4. Procédé selon l'une des revendications 1 à 3, **caractérisé** en ce qu'on utilise, pour déterminer la teneur en alcool et/ou la valeur calorifique, le temps de charge et/ou le temps de décharge d'un condensateur formé dans la cellule de mesure, condensateur dont le diélectrique est le carburant.

5. Procédé selon l'une des revendications 1 à 4, **caractérisé** en ce que les grandeurs électriquement mesurables sont mesurées successivement dans le temps.

6. Procédé selon la revendication 5, **caractérisé** en ce qu'on utilise successivement dans le temps une électrode commune pour la mesure de la constante diélectrique ou encore de la capacitance et pour la mesure de la conductance.

7. Procédé selon l'une des revendications 1 à 6, **caractérisé** en ce que la mesure des grandeurs électriquement mesurables s'effectue dans un montage qui est disposé au voisinage immédiat de la cellule de mesure, tandis que la poursuite du traitement des signaux délivrés par le montage de mesure s'effectue dans un autre montage plus éloigné.

8. Procédé selon la revendication 7, **caractérisé** en ce que la totalité ou une partie des signaux produits par le montage de mesure sont réunis en un signal combiné et transmis au montage de poursuite du traitement par l'intermédiaire d'une ligne de signaux par laquelle peut également s'effectuer l'alimentation électrique.

9. Procédé selon l'une des revendications 1 à 8, **caractérisé** en ce que la mesure de la capacitance et de la conductance du carburant s'effectue au moyen d'électrodes communes, mais on utilise des montages de mesure différents qui, par l'emploi de fréquences très différentes, ne s'influencent pas réciproquement.

10. Procédé selon l'une des revendications 1 à 9, **caractérisé** en ce que la mesure de la capacitance du carburant s'effectue par un circuit oscillant LC (inductance-capacité) dont l'amortissement est compensé en fonction de la valeur de la conductance.

11. Procédé selon la revendication 10, **caractérisé** en ce qu'au lieu d'une inductance classique, on simule la valeur L par un montage électronique.

12. Procédé selon l'une des revendications 1 à 11, **caractérisé** en ce qu'en cas d'utilisation de tension alternative dans le processus de mesure, la fréquence de mesure est maintenue constante tandis que la capacitance varie.

13. Procédé selon l'une des revendications 1 à 12, **caractérisé** en ce que la combinaison des grandeurs s'effectue dans un calculateur programmable (processeur), et en ce qu'on imprime à au moins un processeur un paramètre nécessaire pour l'application respective, par le fait qu'on règle au moins une résistance dont le réglage est lu par un processeur par l'intermédiaire d'un convertisseur analogique-numérique.

14. Procédé selon la revendication 13, **caractérisé** en ce qu'on utilise au moins un paramètre pour la compensation de tolérances de série.

15. Procédé selon la revendication 13, **caractérisé** en ce qu'on utilise au moins un paramètre pour l'adaptation à des applications différentes.

16. Procédé selon la revendication 13, **caractérisé** en ce qu'on utilise au moins un paramètre pour l'identification du montage, par exemple le numéro de série, le numéro de lot, le type de produit ou la protection logicielle.

17. Procédé selon l'une des revendications 13 à 16, **caractérisé** en ce qu'au moins un paramètre est traité par formule.
